# EUROPÄISCHE PATENTSCHRIFT

(11) **EP 2 962 636 B1**
(45) Veröffentlichungstag und Bekanntmachung des Hinweises auf die Patenterteilung: **17.08.2016**
(21) Anmeldenummer: 14175648.6
(22) Anmeldetag: 03.07.2014
(51) Int. Cl.: A61B 5/05, A61B 5/053

(54) **Vorrichtung zur Durchleuchtung eines menschlichen oder tierischen Körpers**
Device for transillumination of a human or animal body
Dispositif de transillumination d'un corps humain ou animal

(43) Veröffentlichungstag der Anmeldung: 06.01.2016
(73) Patentinhaber: Kefenbaum, Arno, 58339 Breckerfeld (DE)
(72) Erfinder: Kefenbaum, Arno, 58339 Breckerfeld (DE)
(74) Vertreter: Patentanwälte Dörner & Kötter PartG mbB

(56) Entgegenhaltungen:
- DE-A1-102004 007 455
- US-A1- 2009 129 652
- GOLDSMITH P F ET AL: "FOCAL PLANE IMAGING SYSTEMS FOR MILLIMETER WAVELENGTHS", IEEE TRANSACTIONS ON MICROWAVE THEORY AND TECHNIQUES, IEEE SERVICE CENTER, PISCATAWAY, NJ, US, Bd. 41, Nr. 10, 1. Oktober 1993 (1993-10-01), Seiten 1664-1674, XP000414462, ISSN: 0018-9480, DOI: 10.1109/22.247910

## Beschreibung

Die Erfindung betrifft eine Vorrichtung zur Durchleuchtung eines menschlichen oder tierischen Körpers zur Abbildung von inneren Organen und/oder Knochen nach dem Oberbegriff des Patentanspruchs 1.

In der medizinischen Diagnostik spielt die Durchleuchtung des menschlichen oder tierischen Körpers zur Abbildung innerer Organe bzw. von Knochen eine große Rolle. Am häufigsten kommt hierbei die konventionelle Röntgendiagnostik zum Einsatz. Hierzu werden Röntgenstrahlen in einer Röntgenröhre erzeugt und auf die zu untersuchende Region des Körpers geleitet. Bei der Durchdringung des Körpers werden die Röntgenstrahlen aufgrund der unterschiedlichen Dichte verschiedener Gewebe bzw. Knochen unterschiedlich stark abgeschwächt. Diese Abschwächung findet als Absorption der Röntgenstrahlen statt. Dichtere Strukturen des Körpers, wie beispielsweise die Knochen, weisen eine höhere Absorption auf, als beispielsweise viel Luft enthaltende Organe, wie das Lungengewebe. Nach Durchdringen des Körpers werden die Röntgenstrahlen mittels unterschiedlicher Detektoren nachgewiesen und in ein Bild umgerechnet. Die hierzu über Jahrzehnte eingesetzten Röntgenfilme wurden dabei in letzter Zeit zunehmend durch Festkörperdetektoren sowie auch durch Speicherfoliensysteme abgelöst. Diese neuen Detektorsysteme ermöglichen die Erzeugung digitaler Bilder, welche anschließend weiterbearbeitet sowie auf Computer-Bildschirmen dargestellt werden können.

Nachteilig an der vorgenannten Röntgendiagnostik ist, dass die zum Einsatz kommende Röntgenstrahlung ionisierend ist. Durch die ionisierende Strahlung können Veränderungen im lebenden Organismus hervorgerufen werden, wodurch Schäden bis hin zu Krebs verursacht sein können. Insbesondere bei Schwangeren wird empfohlen, die Röntgendiagnostik weitgehend zu vermeiden, da das ungeborene Kind auf die ionisierenden Strahlen sehr empfindlich reagiert. In der US 2009/0129652 A1 wird hierzu ein hochauflösendes RF-Bildgebungssystem vorgeschlagen.

Hier will die Erfindung Abhilfe schaffen. Der Erfindung liegt die Aufgabe zu Grunde, eine Vorrichtung sowie ein Verfahren zur Durchleuchtung eines menschlichen oder tierischen Körpers zur Abbildung von inneren Organen und/oder Knochen bereitzustellen, bei der die bei der Röntgendiagnostik vorliegende Gesundheitsgefährdung durch ionisierende Strahlung vermieden ist. Gemäß der Erfindung wird diese Aufgabe durch die Merkmale des kennzeichnenden Teils des Patentanspruchs 1 gelöst.

Mit der Erfindung ist eine Vorrichtung zur Durchleuchtung eines menschlichen oder tierischen Körpers zur Abbildung von inneren Organen und/oder Knochen bereitgestellt, bei der die bei der Röntgendiagnostik vorliegende Gesundheitsgefährdung durch ionisierende Strahlung vermieden ist. Erfindungsgemäß weist die Sendeeinheit ein Array aus matrixförmig angeordneten Sendeantennen und die Empfangseinheit ein Array aus matrixförmig angeordneten Empfangsantennen auf, wobei die Frequenz der von den Sendeantennen emittierbaren Wellen im Bereich von 60 - 300 GHz angesiedelt ist. Die Sendeantennen der Sendeeinheit sind mit einer Steuereinheit verbunden, über die die Sendeantennen zumindest gruppenweise zeitlich versetzt anregbar sind. Die Empfangsantennen sind mit einem Auswertemodul verbunden, das eingerichtet ist, die jeweils von den Empfangsantennen empfangenen Wellen einer Sendeantenne bzw. einer Gruppe von Sendeantennen jeweils bildlich darzustellen und die einzelnen Bilder unter gleichzeitiger Rauschdämpfung und Kontrasterhöhung mittels eines Schärfungsverfahrens zu überlagern. Überraschend hat sich gezeigt, das durch elektromagnetische Wellen im Bereich von 60-300 GHz in Verbindung mit der massiven Überabtastung durch die vorstehend beschriebene Anordnung von Sendeeinheit und Empfangseinheit eine gute Abbildung von inneren Organen sowie auch von Knochen erzielbar ist. Im Gegensatz zur Röntgenstrahlung wird hierbei keine ionisierende Strahlung erzeugt.

In weiterer Ausgestaltung der Erfindung ist die Steuereinheit eingerichtet, die jeweils angeregten Sendeantennen dem Auswertemodul zur Synchronisation zu übermitteln. Hierdurch ist eine hohe Genauigkeit des abgebildeten Organs bzw. Knochens ermöglicht.

In Weiterbildung der Erfindung ist die Steuereinheit mit dem Auswertemodul verbunden, wobei die Auswertung der von den Empfangsantennen empfangenen Wellen auf Basis von der Steuereinheit an das Auswertemodul gelieferten Sendeinformationen erfolgt. Hierdurch ist eine Synchronisation der von den Empfangsantennen empfangenen Wellen zu den von jeweils einer Sendeantenne gesendeten Signale ermöglicht.

In Ausgestaltung der Erfindung ist die Auswerteeinheit eingerichtet, mit Hilfe der Silhouettenschnitt-Dämpfung die überlagerten Bilder in ein dreidimensionales Bild zu überführen. Dieses im Stand der Technik bekannte Standardverfahren bezeichnet ein Verfahren, dass die Gewinnung einer digitalen dreidimensionalen Rekonstruktion einer beliebigen Objektform aus digitalen Bildern ermöglicht. Dabei werden über mindestens zwei Achsen Bilder aus vorgegebenen Positionen digital rund um das Objekt aufgenommen. Nachfolgend werden die erfassten Objekte ähnlich einem Scherenschnitt freigestellt und durch Segmentierung der Eingangsbilder aus Objektsilhouetten die Objektform rekonstruiert.

In weiterer Ausgestaltung der Erfindung ist die Steuereinheit eingerichtet, einzelne Wellenimpulse mit einer Länge über die jeweilige Sendeantenne bzw. Gruppe von Sendeantennen zu senden, die sich aus dem Quotienten aus der gewünschten Bildrate und der Anzahl an Sendeimpulsen bzw. der Anzahl der sendenden Antennen errechnet. Hierdurch ist eine gute Auflösung des darzustellenden Bildes bei vertretbarem Rechenaufwand ermöglicht.

Die gestellte Aufgabe wird weiterhin durch ein Verfahren zur Durchleuchtung eines menschlichen oder tierischen Körpers zur Abbildung von inneren Organen und/oder Knochen mit den Merkmalen des Patentanspruchs 7 gelöst. Hierbei wird zunächst eine Sendeeinheit mit einer Matrix von Sendeantennen auf einer Seite des Körpers und eine Empfangseinheit mit einer Matrix von Empfangsantennen auf der anderen Seite des Körpers angeordnet. Nachfolgend werden - beispielsweise mit Hilfe des Zeilensprungverfahrens oder einem anderen zur Synchronisation von Steuereinheit und Auswertemodul geeigneten Verfahren - hintereinander die einzelnen Sendeantennen mit definierter Leistung angeregt, wodurch Wellenimpulse mit einer Frequenz im Bereich von 60 - 300 GHz ausgesendet werden und wobei das von jeden Wellenimpuls von jeder Empfangsantenne der Empfangseinheit empfangene Signal in eine bildliche Darstellung überführt wird. Die durch jeden Wellenimpuls erzielten bildlichen Darstellungen werden unter gleichzeitiger Rauschdämpfung und Kontrasterhöhung mittels eines Schärfungsverfahrens überlagert.

In Weiterbildung der Erfindung wird bei der Überlagerung der einzelnen Bilder mithilfe der Silhouettenschnitt-Dämpfung ein dreidimensionales Bild erzeugt. Hierdurch ist eine verbesserte Diagnostik ermöglicht. Vorteilhaft beträgt die Länge der von den Sendeantennen emittierten Wellenimpulse maximal 25 Mikrosekunden.

In weiterer Ausgestaltung der Erfindung weisen die Wellenimpulse eine außerhalb des ISM-Bandes auf. Als ISM (Industrial and Scientific Medical)-Bänder werden Frequenzbereiche bezeichnet, die durch Hochfrequenz-Geräte in Industrie, Wissenschaft, Medizin, in häuslichen und ähnlichen Bereichen genutzt werden können. Die ISM-Bänder sind in der Vollzugsordnung für den Funkdienst (VO Funk) in den Artikeln 5.138 und 5.150 weltweit geregelt. Durch die Vermeidung von Frequenzen innerhalb des ISM-Bandes sind Wasserresonanz-Frequenzen vermieden, wodurch hierdurch verursachte übermäßige Signaldämpfungen vermieden sind.

Andere Weiterbildungen und Ausgestaltungen der Erfindung sind in den übrigen Unteransprüchen angegeben. Ein Ausführungsbeispiel der Erfindung ist in den Zeichnungen dargestellt und wird nachfolgend im Einzelnen beschrieben. Es zeigen:
- Figur 1: die schematische Darstellung einer Vorrichtung zur Durchleuchtung eines menschlichen oder tierischen Körpers und
- Figur 2: die schematische Darstellung des Empfangs eines Signals von einer Sendeantenne durch alle Empfangsantennen des Arrays der Empfangseinheit der Vorrichtung aus Figur 1.

Die als Ausführungsbeispiel gewählte Vorrichtung besteht im Wesentlichen aus einer Sendeeinheit 1 sowie einer Empfangseinheit 4, welche einander gegenüberliegend angeordnet sind. Die Sendeeinheit 1 umfasst ein Array aus matrixförmig angeordneten Sendeantennen 2. Die Empfangseinheit 4 umfasst ein Array aus matrixförmig angeordneten Empfangsantennen 5. Die Arrays von Sendeeinheit 1 und Empfangseinheit 4 sind dabei identisch ausgebildet, d.h. die Anzahl der Matrixfelder an Sendeantennen 2 der Sendeeinheit 1 ist gleich der Anzahl der matrixförmig angeordneten Empfangsantennen 5 der Empfangseinheit 4. Die Sendeantennen 2 der Sendeeinheit 1 sind mit einer Steuereinheit 3 verbunden, die derart eingerichtet ist, dass die einzelnen Sendeantennen 2 zeitlich versetzt zueinander anregbar sind. Die Empfangseinheit 5 ist mit einem Auswertemodul 6 verbunden, das eingerichtet ist, die jeweils von den einzelnen Empfangsantennen 5 empfangenen Wellen einer Sendeantennen 2 bildlich darzustellen und die einzelnen Bilder unter gleichzeitiger Rauschdämpfung und Kontrasterhöhung mittels eines Schärfungsverfahrens zu überlagern. Das Auswertemodul 6 ist hierzu mit der Steuereinheit 3 verbunden, wobei die Auswertung der von den Empfangsantennen 5 empfangenen Wellen auf Basis von der Steuereinheit 3 an das Auswertemodul 6 gelieferten Sendeinformationen erfolgt. Das Auswertemodul 6 ist im Ausführungsbeispiel mit einem Bildschirm 7 verbunden, auf dem die von dem Auswertemodul 6 errechneten Bilder grafisch darstellbar sind.

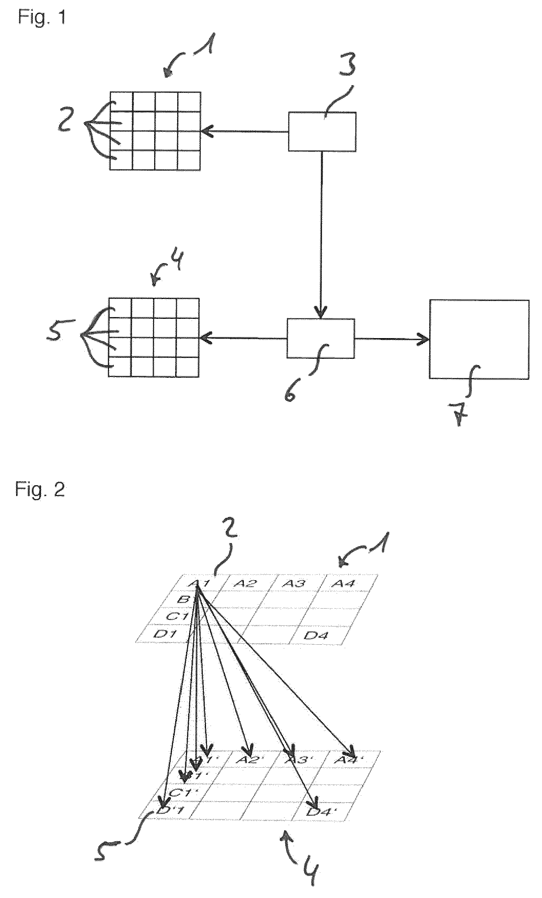

Zur Durchleuchtung eines menschlichen oder tierischen Körpers wird dieser zwischen der Sendeeinheit 1 und Empfangseinheit 4 positioniert, so dass die Matrix von Sendeantennen 2 als Fläche über den Körper und die Matrix von Empfangsantennen als Fläche unter dem Körper angeordnet ist. Die Anzahl der Matrixfelder der Sendeeinheit bzw. der Empfangseinheit beschreibt die Bruttoauflösung der erfindungsgemäßen Vorrichtung im Zweidimensionalen. Mit Hilfe des Zeilensprungverfahrens werden hintereinander die einzelnen Sendeantennen 2, die in Figur 2 entsprechend ihrer Position in der Matrix mit A1, A2,..., An sowie B1, B2,..., An beschriftet sind, mit einer definierten Leistung angeregt. Dabei empfangen die Empfangsantennen 5 der Empfangseinheit 4 alle jeweils ein Signal der von den jeweiligen Sendeantennen 2 emittierten Welle. Hierdurch ist jeweils durch die Auswerteeinheit 6 ein zweidimensionales Bild erzielt. Bei Überlagerung dieser Bilder ergibt sich mit Hilfe der Silhouettenschnitt-Dämpfung durch die Auswerteeinheit 6 ein dreidimensionales Bild, welches auf dem Ausgabebildschirm 7 darstellbar ist.

Im Ausführungsbeispiel ist die Sendeeinheit 1 durch eine Antennenplatte gebildet, welche die Matrix der Sendeantennen 2 umfasst, welche aus 200 x 200 Sendeantennen 2 gebildet ist. Die Steuereinheit 3 umfasst einen HF-Burst Generator, über den elektromagnetische Wellen mit einer Frequenz von 60-300 GHz erzeugt werden können, mit einer spektralen Leistungsdichte von ca. 30 dBm pro Hz. Die von der Steuereinheit 3 über die Sendeantennen 2 emittierten Einzelimpulse haben eine maximale Länge von 25 Mikrosekunden. Diese im Ausführungsbeispiel gewählte Länge der Einzelimpulse von 25 Mikrosekunden ergibt sich aus dem Quotienten aus der gewünschten Bildrate und der Anzahl an Sendeimpulsen bzw. der sendenden Antennen.

Die Auswerteeinheit 6 ist mit der Steuereinheit 3 verbunden und mit dieser synchronisiert und weist einen A/D-Wandler zur Amplitudenkonversion der durch die Empfangsantennen 5 empfangenen Wellen auf. Im Ausführungsbeispiel hat der Analog-Digital Wandler eine Auflösung von 16 Bit.

## Patentansprüche

1. Vorrichtung zur Durchleuchtung eines menschlichen oder tierischen Körpers zur Abbildung von inneren Organen und/oder Knochen, umfassend eine Sendeeinheit zur Emission elektromagnetischer Wellen und eine Empfangseinheit zur Empfang dieser elektromagnetischen Wellen, wobei die Empfangseinheit zur bildlichen Darstellung der empfangenen Wellen eingerichtet ist, wobei die Sendeeinheit (1) ein Array aus matrixförmig angeordneten Sendeantennen (2) und die Empfangseinheit (4) ein Array aus matrixförmig angeordneten Empfangsantennen (5) aufweisen, welche Sendeantennen (2) mit einer Steuereinheit (3) verbunden sind, über die sie zumindest gruppenweise zeitlich versetzt anregbar sind, wobei die Empfangsantennen (5) mit einem Auswertemodul (6) verbunden sind, wobei die Vorrichtung eingerichtet ist, mittels der Sendeantennen (2) elektromagnetische Wellen im Bereich von 60 bis 300 Gigahertz zu emittieren und wobei das Auswertemodul (6) eingerichtet ist,
die jeweils von den Empfangsantennen (5) empfangenen Wellen einer Sendeantenne (2) bzw. einer Gruppe von Sendeantennen (2) jeweils bildlich darzustellen und die einzelnen Bilder unter gleichzeitiger Rauschdämpfung und Kontrasterhöhung mittels eines Schärfungsverfahrens zu überlagern.

2. Vorrichtung nach Anspruch 1 **dadurch gekennzeichnet, dass** die Steuereinheit (3) eingerichtet ist, die jeweils angeregten Sendeantennen (2) dem Auswertemodul (6) zur Synchronisation zu übermitteln.

3. Vorrichtung nach einem der vorgenannten Ansprüche, **dadurch gekennzeichnet, dass** die Steuereinheit (3) mit dem Auswertemodul (6) verbunden ist, wobei die Auswertung der von den Empfangsantennen (5) empfangenen Wellen auf Basis von der Steuereinheit (3) an das Auswertemodul (6) gelieferten Sendeinformationen erfolgt.

4. Vorrichtung nach einem der vorgenannten Ansprüche, **dadurch gekennzeichnet, dass** die Auswerteeinheit (6) eingerichtet ist, mit Hilfe der Siluettenschnitt-Dämpfung die überlagerten Bilder in ein dreidimensionales Bild zu überführen.

5. Vorrichtung nach einem der vorgenannten Ansprüche, **dadurch gekennzeichnet, dass** die Steuereinheit (3) eingerichtet ist, einzelne Wellenimpulse mit einer Länge über die jeweilige Sendeantenne (2) bzw. Gruppe von Sendeantennen (2) zu senden, die sich aus dem Quotienten aus der gewünschten Bildrate und der Anzahl an Sendeimpulsen bzw. der Anzahl der sendenden Antennen errechnet.

6. Verfahren zur Durchleuchtung eines menschlichen oder tierischen Körpers zur Abbildung von inneren Organen und/oder Knochen, wobei eine Sendeeinheit (1) mit einer Matrix von Sendeantennen (2) auf eine Seite des Körpers und eine Empfangseinheit (4) mit einer Matrix von Empfangsantennen (5) auf der anderen Seite des Körpers angeordnet wird, wobei nachfolgend mit Hilfe des Zeilensprungverfahrens hintereinander die einzelnen Sendeantennen (2) mit konstanter Leistung angeregt werden, wodurch Wellenimpulse mit einer Frequenz im Bereich von 60 bis 300 Gigahertz ausgesendet werden, wobei das von jeden Wellenimpuls von jeder Empfangsantenne (5) der Empfangseinheit (4) empfangene Signal in eine bildliche Darstellung überführt wird und die durch jeden Wellenimpuls erzielten bildlichen Darstellungen unter gleichzeitiger Rauschdämpfung und Kontrasterhöhung mittels eines Schärfungsverfahrens überlagerten werden.

7. Verfahren nach Anspruch 6, **dadurch gekennzeichnet, dass** bei der Überlagerung der einzelnen Bilder mit Hilfe der Siluettenschnitt-Dämpfung ein dreidimensionales Bild erzeugt wird.

8. Verfahren nach Anspruch 6 oder 7 **dadurch gekennzeichnet, dass** die Länge der von den Sendeantennen (2) emittierten Wellenimpulse durch den Quotienten aus der gewünschten Bildrate und der Anzahl an Sendeimpulsen bzw. der Anzahl der sendenden Antennen ermittelt wird.

9. Verfahren nach einem der Ansprüche 6 bis 8, **dadurch gekennzeichnet, dass**
die von einer Sendeantenne (2) emittierten Wellenimpulse eine Frequenz außerhalb des ISM-Bandes aufweisen.

## Claims

1. Device for transillumination of a human or animal body for imaging of internal organs and/or bones comprising a transmission unit for emitting electromagnetic waves and a receiving unit for receiving these electromagnetic waves, wherein the receiving unit is adapted for visual representation of the received waves, wherein the transmission unit (1) has an array of transmitting antennas (2) arranged in matrix form and the receiving unit (4) has an array of receiving antennas (5) arranged in matrix form, which transmitting antennas (2) are connected to a control unit (3), via which they are excitable at least temporally offset in groups, wherein the receiving antennas (5) are connected to an evaluation module (6), wherein the device is adapted to emit electromagnetic waves in the range of 60 to 300 gigahertz by means of the transmitting antennas (2) and wherein the evaluation module (6) is adapted to visually represent the waves of a transmitting antenna (2) or a group of transmitting antennas (2) respectively received by the receiving antennas (5) and to superimpose the individual images with simultaneous noise attenuation and contrast enhancement by means of a sharpening method.

2. Device according to claim 1, **characterised in that** the control unit (3) is adapted to transmit the respectively excited transmitting antennas (2) to the evaluation module (6) for synchronisation.

3. Device according to one of the previous claims, **characterised in that** the control unit (3) is connected to the evaluation module (6), wherein the evaluation of the waves received by the receiving antennas (5) is carried out on the basis of the transmitted information provided to the evaluation module (6) from the control unit (3).

4. Device according to one of the previous claims, **characterised in that** the evaluation unit (6) is adapted to transform the superimposed images into a three-dimensional image by means of silhouette intersection attenuation.

5. Device according to one of the previous claims, **characterised in that** the control unit (3) is adapted to transmit individual wave pulses with a length calculated from the quotient of the desired image rate and the number of transmission pulses or the number of the transmitting antennas via the respective transmitting antenna (2) or group of transmitting antennas (2).

6. Method for transillumination of a human or animal body for imaging of internal organs and/or bones, wherein a transmission unit (1) with a matrix of transmitting antennas (2) is arranged on one side of the body and a receiving unit (4) with a matrix of receiving antennas (5) is arranged on the other side of the body, wherein subsequently the individual transmitting antennas (2) are successively excited with constant power by means of interlaced scanning, whereby wave pulses are transmitted with a frequency in the range of 60 to 300 gigahertz, wherein the signal of each wave pulse received by each receiving antenna (5) of the receiving unit (4) is transformed into a visual representation and the visual representations achieved by each wave pulse are superimposed with simultaneous noise attenuation and contrast enhancement by means of a sharpening method.

7. Method according to claim 6, **characterised in that** a three-dimensional image is created during superimposition of the individual images by means of the silhouette intersection attenuation.

8. Method according to claim 6 or 7, **characterised in that** the length of the wave pulses emitted by the transmitting antennas (2) is determined by the quotient of the desired image rate and the number of transmission pulses or the number of transmitting antennas.

9. Method according to one of claims 6 to 8, **characterised in that** the wave pulses emitted by a transmitting antenna (2) have a frequency out of the ISM band.

## Revendications

1. Dispositif de transillumination d'un corps humain ou animal pour obtenir l'image d'organes internes et/ou d'os, comprenant une unité émettrice d'ondes électromagnétiques et une unité réceptrice de ces ondes électromagnétiques, sachant que l'unité réceptrice est configurée pour représenter les ondes reçues sous forme d'image, sachant que l'unité émettrice (1) présente un ensemble d'antennes émettrices (2) agencées en forme de matrice et que l'unité réceptrice (4) présente un ensemble d'antennes réceptrices (5) agencées en forme de matrice, lesquelles antennes émettrices (2) sont reliées à une unité de commande (3) via laquelle elles sont excitables au moins par groupes avec décalage dans le temps, sachant que les antennes réceptrices (5) sont reliées à un module d'analyse (6), sachant que le dispositif est configuré pour émettre des ondes électromagnétiques dans la bande des 60 à 300 gigahertz au moyen des antennes émettrices (2), et sachant que le module d'analyse (6) est configuré pour représenter sous forme d'images respectives les ondes reçues par les antennes réceptrices (5) d'une antenne émettrice (2) et/ou d'un groupe d'antennes émettrices (2), et de superposer les différentes images tout en atténuant simultanément le bruit et en haussant le contraste au moyen d'un procédé d'amélioration de la netteté.

2. Dispositif selon la revendication 1, **caractérisé en ce que** l'unité de commande (3) est configurée pour communiquer au module d'analyse (6), à des fins de synchronisation, les antennes émettrices (2) respectivement excitées.

3. Dispositif selon l'une des revendications précédentes, **caractérisé en ce que** l'unité de commande (3) est reliée au module d'analyse (6), sachant que l'analyse des ondes reçues par les antennes réceptrices (5) a lieu sur la base des informations d'émission livrées au module d'analyse (6) par l'unité de commande (3).

4. Dispositif selon l'une des revendications précédentes, **caractérisé en ce que** l'unité d'analyse (6) est configurée pour, à l'aide de l'atténuation de la section de silhouette, transférer les images superposées vers une image tridimensionnelle.

5. Dispositif selon l'une des revendications précédentes, **caractérisé en ce que** l'unité de commande (3) est configurée pour émettre via l'antenne émettrice respective (2) et/ou le groupe d'antennes réceptrices (2) respectif, des impulsions d'onde individuelles dont la longueur résulte du quotient formé avec le débit d'image souhaité et le nombre d'impulsions d'émission et/ou le nombre d'antennes en train d'émettre.

6. Procédé de transillumination d'un corps humain ou animal pour reproduire les organes intérieurs et/ou les os, sachant qu'une unité émettrice (1) comportant une matrice d'antennes émettrices (2) est agencée d'un côté du corps et une unité réceptrice (4) avec une matrice d'antennes réceptrices (5) est agencée de l'autre côté du corps, sachant qu'ensuite, à l'aide du procédé de saut de lignes, les différentes antennes émettrices (2) sont excitées les unes après les autres à une puissance constante, faisant que sont émises des impulsions d'ondes à une fréquence située dans la bande des 60 à 300 gigahertz, sachant que chaque signal reçu de chaque impulsion d'onde de chaque antenne réceptrice (5) de l'unité réceptrice (4) est transformé en une représentation imagée et que les représentations imagées obtenues par chaque impulsion d'onde sont superposées en atténuant simultanément le bruit et en haussant le contraste au moyen d'un procédé d'amélioration de la netteté.

7. Procédé selon la revendication 6, **caractérisé en ce qu'**une image tridimensionnelle est générée lors de la superposition des images individuelles à l'aide d'une atténuation de la section de silhouette.

8. Procédé selon la revendication 6 ou 7, **caractérisé en ce que** la longueur des impulsions d'onde émises par les antennes émettrices (2) est déterminée par le quotient formé avec le débit d'image souhaité et le nombre d'impulsions émises et/ou le nombre d'antennes émettrices.

9. Procédé selon l'une des revendications 2 à 8, **caractérisé en ce que** les impulsions d'ondes émises par une antenne émettrice (2) présentent une fréquence située hors de la bande ISM.
